# EUROPEAN PATENT APPLICATION

(11) **EP 4 755 307 A1**
(43) Date of publication of application: **10.06.2026**
(21) Application number: 24217466.2
(22) Date of filing: 04.12.2024
(51) Int. Cl.: A61B 6/03, A61B 6/06, A61B 6/51

(54) **COMPUTER-IMPLEMENTED METHOD OF GENERATING A PANORAMIC IMAGE OF A PATIENT'S ORAL CAVITY**

(71) Applicant: Dentsply Sirona Inc., York, PA 17401 (US); SIRONA Dental Systems GmbH, 64625 Bensheim (DE)
(72) Inventor: Eichner, Stefan, 64625 Bensheim (DE); Jollans, Thomas, 64625 Bensheim (DE)
(74) Representative: Aldridge, Henry Alexander

(57) **Abstract**

The present invention relates to a computer implemented method of generating a panoramic image of a patient (P), comprising: a step of defining a focal curve (F) relative to the patient's oral cavity (O), wherein the focal curve (F) follows the shape of the jawbone (1) of the patient (P), wherein the focal curve (F) has a predefined thickness (L) which includes at least the teeth (T) to be imaged; a step of acquiring a plurality of two dimensional x-ray projection images of the patient (P) along the focal curve (F) by means of rotating an x-ray source (3), an x-ray collimator (12) and a flat panel x-ray detector (4) around the patient's head in accordance with a predefined trajectory relative to the patient (P), wherein the x-ray collimator (12) is positioned between the patient's head and the x-ray source (3), wherein the collimator (12) has an x-ray aperture (13) to restrict the x-ray cone beam angle, wherein the width of the x-ray aperture (13) is adjustable for varying the x-ray cone beam angle. In the acquisition step, the width of the aperture (13) is continuously varied such that the width of the aperture (13) used to expose the molar region (M) of the focal curve (F) is larger than the width of the aperture (13) used to expose to the incisal region (I); and in the acquisition step, the readout region (R) of the x-ray detector (4) is continuously varied in the horizontal direction such that the readout region (R) of the x-ray detector (4) is equal to or larger that the region of the x-ray detector (4) being actively exposed through the aperture (13).

## Description

### TECHNICAL FIELD OF THE INVENTION

The present invention relates to a computer-assisted x-ray system and a computer-implemented method of generating a panoramic image of the dental condition of a patient.

### BACKGROUND ART OF THE INVENTION

An orthopantomogram (OPG), generally known as panoramic image is the standard procedure for a dentist to get a quick dental overview of the patient. The panoramic image usually shows all teeth of the upper and lower jaw as well as the temporomandibular joints and part of the eye sockets. In general, for each available panoramic program in an extraoral x-ray device, there is a predefined trajectory that describes the trajectory of the x-ray source and the x-ray detector around the patient's head during imaging. During the imaging, a plurality of two-dimensional x-ray projection images are acquired by rotating the x-ray detector and the x-ray source along the trajectory around the patient's head. This trajectory initially serves to determine the position of the focal curve in the patient's jaw, which defines the dental structures to be sharply imaged within the panoramic image. In addition, the trajectory also serves to determine the layer thickness, which has a direct influence on the representation of the objects within the panoramic image and is a measure of the local blurring of the objects outside the focal curve. Thus, the focal curve has a layer thickness which includes at least the teeth to be imaged. The panoramic image is reconstructed based on the focal curve, and the plurality of 2D x-ray projection images. If the layer thickness is too wide, this reduces the ability to sharply recognize individual dental structures and also the possibilities for optimizing the panoramic image using know autofocus methods. This makes diagnosis more difficult in the affected areas of the panoramic image. Furthermore, the trajectory also serves to determine the local radiation area through the patient's jaw during rotation of the x-ray source and the x-ray detector around the patient's head, which, however, is only orthoradial to the tooth plane in a few areas. This leads to unfavorable overlapping of the teeth within the reconstructed panoramic image, which in turn makes diagnosis more difficult in the affected regions of the panoramic image. Conventional extraoral x-ray systems, which exhibit both of these problems, use either a dedicated panoramic detector with a narrow detector width or a flat-panel detector with a narrow partial readout region at a fixed, predefined detector position during the rotation for panoramic imaging.

### DISCLOSURE OF THE INVENTION

By using a flat-panel detector with a variable readout-region and the capability of a temporally and spatially variable aperture control, both problems can be addressed individually or together. The knowledge of the recording geometry of the raw 2D x-ray projection images recorded during the rotation enables not only the reconstruction of the predefined layer which results from the trajectory specific focal curve, but also the reconstruction of additional layers and viewing angles.

An objective of the invention is to improve the diagnostic accuracy and image quality of panoramic images taken with extra-oral x-ray systems.

This objective is achieved by the method as defined in claim 1, and the x-ray system as defined in claim 10. The subject-matters of the dependent claims define further developments and preferred embodiments.

The method according to the present invention is a computer-implemented method and is used to generate a panoramic image of the dental condition of a patient. The method comprises: a step of defining a focal curve relative to the patient's oral cavity, wherein the focal curve follows the shape of the jawbone of the patient, wherein the focal curve has a predefined thickness which includes at least the teeth to be imaged; a step of acquiring a plurality of two-dimensional x-ray projection images (hereinafter referred to as x-ray images) of the patient along the focal curve by means of rotating an x-ray source, an x-ray collimator and a flat-panel x-ray detector around the patient's head in accordance with a predefined trajectory relative to the patient, wherein the x-ray collimator is positioned between the patient's head and the x-ray source, wherein the collimator has an x-ray aperture to restrict the x-ray cone beam angle from the x-ray source, wherein the width of the x-ray aperture is adjustable for varying the x-ray cone beam angle. In the acquisition step, the width of the aperture is continuously varied such that the width of the aperture used to expose the molar region of the focal curve is larger than the width of the aperture used to expose the incisal region. And in the acquisition step, the readout region of the x-ray detector is continuously varied in the horizontal direction (i.e., extending in the occlusal plane) such that the readout region of the x-ray detector is equal to or larger than the region of the x-ray detector being exposed through the aperture.

According to the present invention it is possible to use a variable x-ray detector readout region width and a variable x-ray detector readout region position in combination with synchronous variable x-ray collimation control. Namely, the width as well as the center position of the read-out region relative to the flat-panel x-ray detector can be continually varied during the acquisition. The readout region of the x-ray detector is kept synchronously equal to or larger than the region of the x-ray detector being exposed through the aperture. For the latter case, the dark current recorded on the x-ray detector can be used for e.g., artefact correction purposes. The variable collimation control also allows to adapt the x-ray dose for the patient.

According to the present invention, the variable readout region width of the flat-panel x-ray detector can be used to compensate during rotation for changes in the trajectory specific layer thickness. Thereby, in the molar regions which are larger than the incisal regions, comparatively more x-ray data can be collected, which in turn improves the visibility/diagnosis of dental regions as well as the possibilities to use of commonly known autofocus methods for local optimization. Thereby, the panoramic image can be reconstructed with same image quality at all regions within the desired layer thickness.

According to an embodiment of present invention, the width of the aperture used to expose the molar region of the focal curve is preferably at least 2 times and maximally 10 times larger than the width of the aperture used to expose the incisal region. The width of the aperture used to expose the incisal region can be predefined according to the geometry of the x-ray device, and standard patient size.

According to an embodiment of present invention, the width of the aperture is varied decreasingly from the molar region towards the incisal region. The decrease of the aperture width is preferably linear.

According to an embodiment of present invention, in the acquisition step, the voltage and/or current of the x-ray source is continuously varied such that the voltage and/or current of the x-ray source when exposing the molar region of the focal curve is smaller than the voltage of the x-ray source when exposing the incisal region. Thereby, the x-ray dose can be preferably kept constant per x-ray image.

According to an embodiment of present invention, in the acquisition step, the frame rate of the x-ray detector is continuously varied such that the frame rate of the x-ray detector used to image the molar region of the focal curve is smaller than the frame rate of the x-ray detector used to image the incisal region.

According to an embodiment of present invention, the frame rate is varied increasingly from the molar region towards the incisal region. The increase of the frame rate is preferably linear.

According to an embodiment of present invention, in the acquisition step, the gain of the x-ray detector is continuously varied such that the gain of the x-ray detector used to image the molar region of the focal curve is different than the gain of the x-ray detector used to image the incisal region. Herein, preferably a high dose is accompanied by a low gain. Preferably a low dose is accompanied by a high gain.

According to an embodiment of present invention, in the acquisition step, the readout region of the x-ray detector is continuously varied between 50 to 500 pixels in the width direction. The flat panel x-ray detector has preferably a native pixel size of 50 to 240 micrometer.

According to an embodiment of present invention, in the acquisition step, the center point of the width of the aperture relative to the readout region of the x-ray detector is continuously varied such that the beam direction passing from the x-ray source to the center point of the redout region remains orthoradial to the focal curve or as close as possible to being orthoradial to the focal curve. Herein the tangent of the focal curve relative to the imaging trajectory is known by the x-ray device.

According to the present invention, the variable readout region position within the flat-panel x-ray detector during rotation allows more dental structures of the dental arch to be captured orthoradially in as much as possible. This leads to a significant reduction in overlapping tooth crowns, which in turn improves diagnostic capabilities. This is a particular advantage for caries detection.

According to the present invention, it is also possible to combine the variable readout region width with the variable readout region position during rotation, which improves the diagnostic possibilities and the quality of the panoramic image resulting from the reconstructions.

According to the present invention, the variable readout region width and the variable readout position for the flat-panel detector serve each as software-based correction techniques. The flat-panel x-ray detector reading process is controlled by a dedicated software. The synchronous control of the collimator, namely the aperture size and aperture position are controlled by a dedicated control means.

The present invention also provides an x-ray system for generating a panoramic image of a patient. The x-ray system comprises: an x-ray source for generating an x-ray cone beam; a flat-panel x-ray detector facing the x-ray source; and an x-ray collimator. The x-ray collimator is positioned between the patient's head and the x-ray source. The collimator has an x-ray aperture to restrict the x-ray cone beam angle. The width and center position of the x-ray aperture is adjustable for varying the x-ray cone beam angle. The adjusting mechanism may have one or more electro-mechanically movable shields made of x-ray absorbing/opaque material. The x-ray system also comprises a control means configured to carry out the above-mentioned computer implemented method.

The present invention also provides a computer program comprising computer-readable codes which, when executed by a computer-assisted x-ray system, causes the system to perform the above-mentioned computer implemented method.

The present invention also provides a computer readable storage means which stores the computer program.

A major advantageous effect of the present invention is that the above-mentioned methods significantly improve the image quality and diagnostic suitability of the resulting panoramic images and avoid the need for repeated recordings. This further eliminates the need for specially designed artifact-reducing trajectories, which in turn simplifies the operation of the extraoral x-ray systems.

### BRIEF DESCRIPTION OF THE DRAWINGS

In the following description, the present invention will be explained in more detail with reference to the exemplary embodiments and with reference to the drawings, wherein
Fig. 1 is a schematic drawing of an x-ray system according to an embodiment of the present invention;
Fig. 2 is a schematic drawing of two different trajectories of the x-ray detector and the x-ray source revolving around the patient's head according to alternative embodiments of the present invention;
Fig. 3 is a partial schematic drawing of the x-ray system according to an embodiment of the present invention, during the molar region is x-ray exposed;
Fig. 4 is a partial schematic drawing of the x-ray system according to an embodiment of the present invention, during the incisal region is x-ray exposed;
Fig. 5 is a schematic drawing of the x-ray collimator of the x-ray system according to an embodiment of the invention;
Fig. 6a is a schematic drawing of a flat-panel x-ray detector and a constant readout region according to the prior art; and
Fig. 6b is a schematic drawing of the flat-panel x-ray detector and a variable readout region according to an embodiment of the present invention.

The reference numbers shown in the drawings designate the elements listed below, which are referred to in the following description of the exemplary embodiments.
1. X-ray system
2. X-ray device
3. X-ray source
4. X-ray detector (Flat Panel Sensor)
5. Operating unit (user interface)
6. Head fixation
7. Bite block
8. Computing unit (Computer)
9. Display device (Screen)
10. Input device (Keyboard)
11. Input device (Mouse)
12. X-ray collimator
13. X-ray aperture
13a. Shields

F: Focal Curve
I: Incisal Region
J: Jawbone
L: Predefined (Layer) Thickness
M: Molar region
O: Oral cavity
P: Patient
R: Readout Region of Flat Panel Sensor
T: Tooth

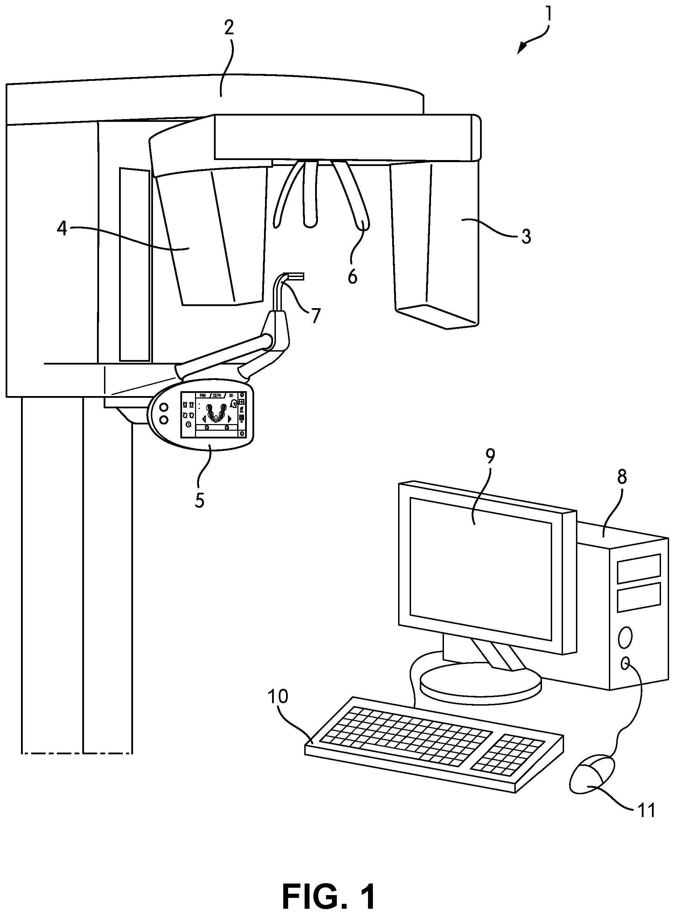

The method according to the present invention, which is explained in detail below, is a computer-implemented method and can be carried out on a computer-assisted x-ray system (1) as shown in an embodiment in Fig. 1.

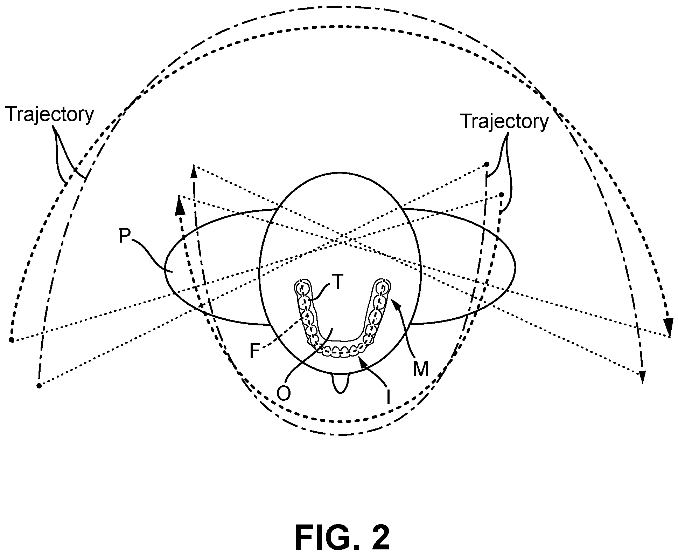

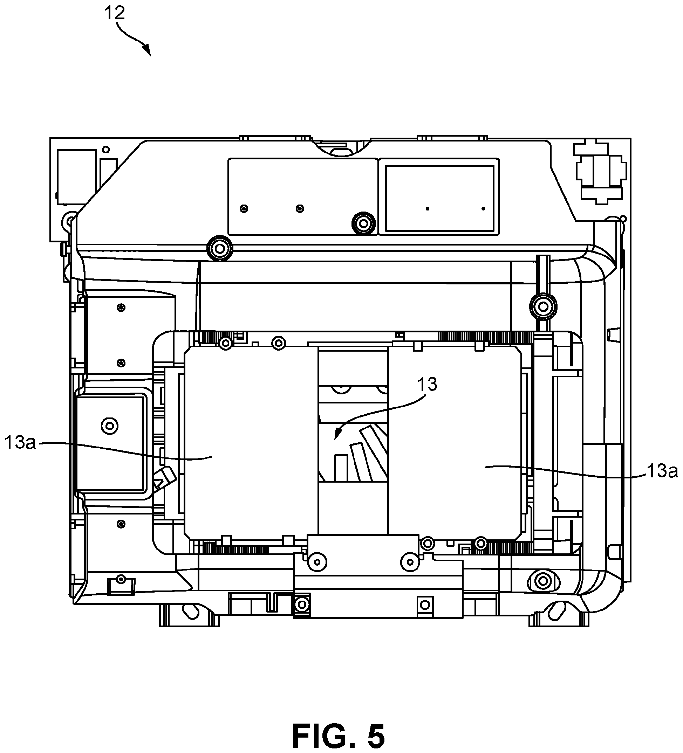

The present invention also includes a corresponding computer program having computer-readable code for implementing the method. The computer program is provided on a computer readable storage medium accessible to the x-ray system (1). The computerized x-ray system (1) comprises an x-ray device (2) for performing patient imaging, which generates 2D x-ray projection images or a sinogram such as a stack of 2D x-ray projection images. The x-ray device (2) has an x-ray source (3) and an x-ray detector (4), specifically a flat panel detector that are rotated around the patient's head during the imaging. The trajectory of the x-ray source (3) and the x-ray detector (4) during the imaging can describe a circular path. However, it can also assume a form deviating from this. Fig 2 shows various example trajectories (see dashed lines). If several actuators (not shown) are controlled simultaneously, a trajectory deviating from a pure circular path around the patient's head can be achieved. The patient's head can be optionally positioned in the x-ray device (2) with a bite block (7) and with a head fixation (6). The trajectory (*course*) of the x-ray source (3) and the x-ray detector (4) with respect to the bite block (7) and the head fixation (6) are known by the x-ray system (1). The x-ray detector (4) detects the x-rays emitted by the x-ray source (3) during the rotation. The x-ray device (2) has an x-ray collimator (12) as shown in Fig. 5. The x-ray collimator (12) is positioned between the patient's head and the x-ray source (3). The x-ray collimator (12) is attached into the housing of the x-ray source (3) so that they move together. The collimator (12) has an x-ray aperture (13) to restrict the x-ray cone beam angle emitted from the x-ray source (3). The width of the x-ray aperture (13) is adjustable for varying the x-ray cone beam angle. The 2D x-ray projection images are acquired, namely read out from the readout regions of x-ray flat panel detector (4) with a predetermined frame rate. This can be set and variably controlled by the x-ray system (1). The computerized x-ray system (1) also comprises an operating unit (5) such as a user interface, a computing unit (8) e.g., a computer which can be connected to the x-ray device (2), and a display device (9) such as a screen for visualizing any data sets i.e., the x-ray projection images, or panoramic images reconstructed through the method. The computer may be connected to the x-ray device (2) via a local area network (not shown) or, alternatively, via the Internet. The computer is connected to input devices such as a keyboard (10), mouse (11), and the like. The computer may be also part of a cloud. Alternatively, the computer may be integrated into the x-ray device (2). Alternatively, all or some of the computations may take place in the cloud as cloud computing. The computer executes the computer program and provides the data sets, e.g., the panoramic images for visualization on the screen. The screen may be provided spatially separate from or integrated with the x-ray device (2). Preferably, the computer may also control all functions of the x-ray device (2) for imaging. Alternatively, separate computers may be used for the control, operation, and panoramic image reconstruction.

The x-ray system (1) is preferably configurable as an IoT system and can be bidirectionally connected via a local area network and/or the internet (not shown) to other dental devices such as optical intraoral scanners for acquiring digital impression of the dental condition of the patient, dental milling machines for manufacturing dental restorations, and additive manufacturing machines such as 3D printers, and for cloud computing, data exchange, remote control, and the like. Since the x-ray system (1) has a flat panel detector (4) it may be optionally used for different imaging modalities such as digital volume (3D) tomography (DVT) imaging or panoramic (PAN) imaging. The operating unit (5) and/or the computer can be used for selecting the modalities and related settings.

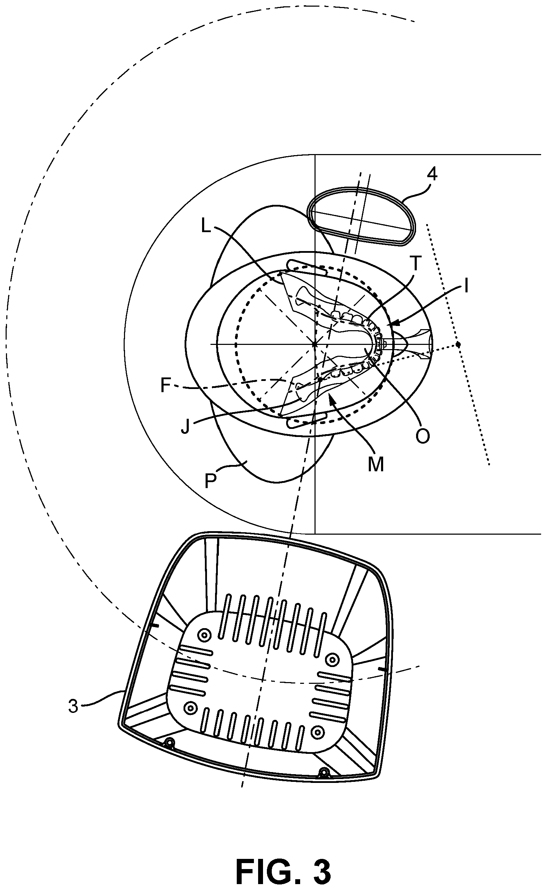

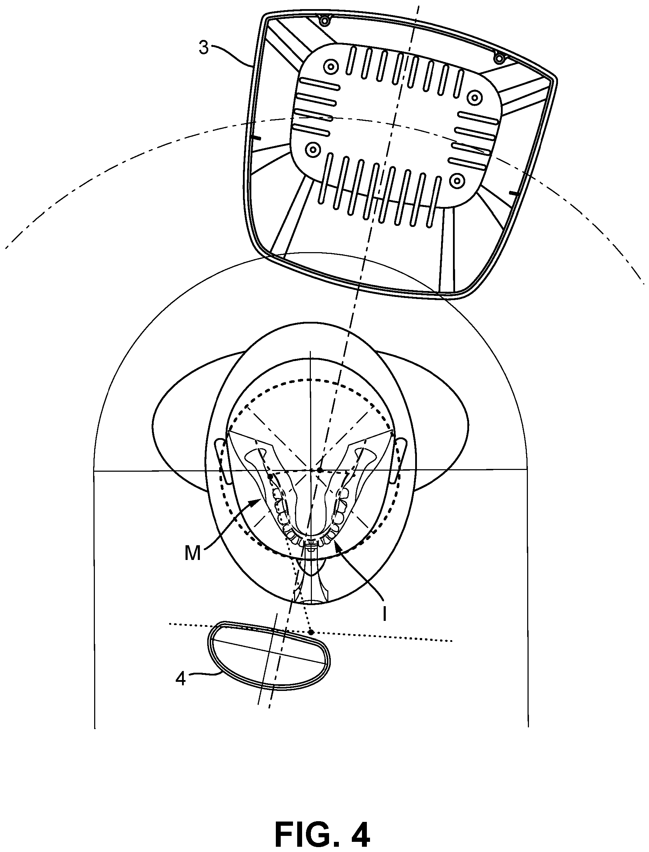

The computer implemented method is for generating a panoramic image of a patient (P), in particular of the dental condition of the patient. The x-ray device (2) has a control means configured to carry out the method. According to the computer implemented method, a focal curve (F) as shown in Fig. 3 and Fig. 4 is defined relative to the patient's oral cavity (O). The focal curve (F) follows the shape of the jawbone (J) of the patient (P). The focal curve (F) has a predefined thickness (L) which includes at least the teeth (T) to be imaged. A plurality of two-dimensional x-ray projection images of the patient (P) along the focal curve (F) are acquired by means of rotating an x-ray source (3), an x-ray collimator (12) and an x-ray detector (4) around the patient's head in accordance with a predefined trajectory relative to the patient (P). The x-ray collimator (12) is positioned between the patient's head and the x-ray source (3). The x-ray collimator (12) has an x-ray aperture (13) as shown in Fig. 5 to restrict the x-ray cone beam angle. The width of the x-ray aperture (13) is adjustable for varying the x-ray cone beam angle. Two shields (13a) are movably arranged to continuously set the width of the aperture (13). The shields (13a) can be made from x-ray absorbing or x-ray opaque material. There are also preferably two movable shields that define the height of the aperture. But these are preferably not changed during rotation.

During the acquisition, the width of the aperture (13) is continuously varied such that the width of the aperture (13) used to expose the molar region (M) of the focal curve (F) is larger than the width of the aperture (13) used to expose to the incisal region (I). Furthermore, during the acquisition, the readout region (R) of the x-ray detector (4) is continuously varied in the horizontal direction such that the readout region (R) of the x-ray detector (4) is equal to or larger that the region of the x-ray detector (4) being actively exposed through the aperture (13). Thereby the amount of x-ray data collected for a particular region can be accordingly matched to the size of the dental structure in that particular region. The x-ray device (2) geometry, i.e., the distance of the x-ray detector (4) and the x-ray source (3) to the focal curve (F) are known. The panoramic image is reconstructed based on the focal curve, the predefined thickness, and the plurality of 2D x-ray projection images acquired.

In an embodiment, the width of the aperture (13) used to expose the molar region (M) of the focal curve (F) is at least 2 times and maximally 10 times larger than the width of the aperture (13) used to expose to the incisal region (I). The width of the aperture (13) used to expose the incisal region (I) can be preset in accordance with the geometry of the x-ray device (2). The geometry of the x-ray device (2) is for example defined through the distances between the patient, namely the bite block and the x-ray detector (4), and x-ray source (3) respectively, as well as the above-mentioned trajectories. The width of the aperture (13) is varied decreasingly from the molar region (M) towards the incisal region (I). The decrease could be realized preferably in a linear manner. Other profiles different than such linear profile, which are specific to the local changes in the dental shape of the patient jaw may be alternatively used. Such a patient specific profile can be obtained from the digital impression of the oral cavity taken by an intraoral scanner (not shown) connected to the x-ray system. The digital impression (3D surface image of the intraoral dental condition) may be also used to determine the position and shape of the focal curve and the predetermined thickness for the dental structure along the focal curve to be sharply imaged.

In an embodiment, in the acquisition step, the voltage and/or current of the x-ray source (3) is continuously varied such that the voltage and/or current of the x-ray source (3) when exposing the molar region (M) of the focal curve (F) is smaller than voltage of the x-ray source (3) when exposing the incisal region (I). In a preferred embodiment, the voltage and/or current of the x-ray source (3) is continuously varied such the x-ray dose remains constant per x-ray projection image.

In an embodiment, in the acquisition step, the frame rate of the x-ray detector (4) is continuously varied such that the frame rate of the x-ray detector (3) used to image the molar region (M) of the focal curve (F) is smaller than the frame rate of the x-ray detector (4) used to image to the incisal region (I). The smallest frame rate is preferably 50 frames per second. The highest frame rate is preferably 300 frames per second.

In an embodiment, the frame rate is varied increasingly from the molar region (M) towards the incisal region (I). The increase in the frame rate is preferably realized in a linear manner.

In an embodiment, in the acquisition step, the gain of the x-ray detector (4) is continuously varied such that the gain of the x-ray detector (4) used to image the molar region (M) of the focal curve (F) is different than the gain of the x-ray detector (4) used to image the incisal region (I). Herein it is preferred that a high dose is associated with a low gain. And more preferably a low dose is associated with a high gain.

In an embodiment, in the acquisition step, the readout region (R) of the x-ray detector (4) is continuously varied in between 50 to 500 pixels in the width direction, wherein the flat panel x-ray detector (4) has a native pixel size of 50 to 240 micrometer.

Flat panel sensor technologies for dental applications are based either on TFT (e.g. amorphous silicon or IGZO) or, depending on the size, on one or more CMOS wafers. Cesium iodide (CsI) is usually used as the scintillator material. They can be used in 1x1 binning for panorama imaging and/or 2x2 binning for DVT. The readout area of the flat-panel detectors can be predefined (so-called partial mode). The larger the area to be read out, the lower the resulting frame rate. The dynamic range is usually 16 bits. Multiple gain factors can be set and selected depending on the application.

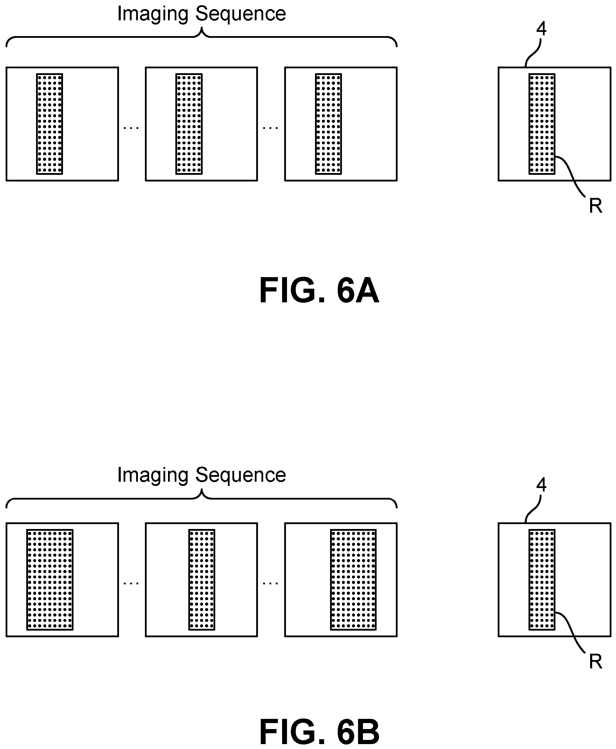

Fig. 6a shows a conventional technique in which the width and position of the readout region (R) of the x-ray detector (4) is constant throughout the acquisition. The width is in the horizonal direction. The dots in the readout region (R) schematically show some pixels to be read out. The three dots among the x-ray detectors (4) indicate that there is a sequence of readouts. For comparison, Fig. 6b shows a technique according to the present invention wherein the width and position of the readout region (R) of the x-ray detector (4) have been varied.

In an embodiment, in the acquisition step, the center point of the width of the x-ray aperture (13) relative to the x-ray detector (4) is continuously varied such that the x-ray beam direction passing from the x-ray source (3) to the center point of the redout region (R) remains orthoradial to the focal curve (F) or as close as possible to being orthoradial to the focal curve (F). In case of orthoradial imaging, said x-ray beam direction becomes perpendicular to the tangential direction of the focal curve. However, this might not be always possible along the focal curve because of the size limitations of the x-ray detector and collimator. Then, said beam direction is aligned as close as possible with the direction perpendicular to the tangential direction of the focal curve.

The present invention also discloses a computer program comprising computer-readable code which, when executed by the computer-assisted x-ray system (1), causes the said system to perform the above-described methods. A computer readable storage means stores the computer program and is accessible to the x-ray system (1).

## Claims

1. A computer implemented method of generating a panoramic image of a patient (P) from a plurality of two-dimensional x-ray projection images of the patient (P), comprising:
a step of defining a focal curve (F) relative to the patient's oral cavity (O), wherein the focal curve (F) follows the shape of the jawbone (J) of the patient (P), wherein the focal curve (F) has a predefined thickness (L) which includes at least the teeth (T) to be imaged;
a step of acquiring the plurality of two-dimensional x-ray projection images of the patient (P) along the focal curve (F) by means of rotating an x-ray source (3), an x-ray collimator (12) and a flat-panel x-ray detector (4) around the patient's head in accordance with a predefined trajectory relative to the patient (P), wherein the x-ray collimator (12) is positioned between the patient's head and the x-ray source (3), wherein the collimator (12) has an x-ray aperture (13) to restrict the x-ray cone beam angle, wherein the width of the x-ray aperture (13) is adjustable for varying the x-ray cone beam angle,
**characterized in that**
in the acquisition step, the width of the aperture (13) is continuously varied such that the width of the aperture (13) used to expose the molar region (M) of the focal curve (F) is larger than the width of the aperture (13) used to expose to the incisal region (I); and
in the acquisition step, the readout region (R) of the x-ray detector (4) is continuously varied in the horizontal direction such that the readout region (R) of the x-ray detector (4) is equal to or larger that the region of the x-ray detector (4) being actively exposed through the aperture (13).

2. The computer implemented method according to claim 1, **characterized in that** the width of the aperture (13) used to expose the molar region (M) of the focal curve (F) is at least 2 times and maximally 10 times larger than the width of the aperture (14) used to expose to the incisal region (I).

3. The computer implemented method according to claim 2, **characterized in that** the width of the aperture (13) is varied decreasingly from the molar region (M) towards the incisal region (I).

4. The computer implemented method according to any one of claims 1 to 3, **characterized in that** in the acquisition step, the voltage and/or current of the x-ray source (3) is continuously varied such that the voltage and/or current of the x-ray source (3) when exposing the molar region (M) of the focal curve (F) is smaller than voltage of the x-ray source (3) when exposing the incisal region (I).

5. The computer implemented method according to any one of claims 1 to 4, **characterized in that** in the acquisition step, the frame rate of the x-ray detector (4) is continuously varied such that the frame rate of the x-ray detector (3) used to image the molar region (M) of the focal curve (F) is smaller than the frame rate of the x-ray detector (4) used to image to the incisal region (I).

6. The computer implemented method according to claim 5, **characterized in that** the frame rate is varied increasingly from the molar region (M) towards the incisal region (I).

7. The computer implemented method according to any one of claims 1 to 6, **characterized in that** in the acquisition step, the gain of the x-ray detector (4) is continuously varied such that the gain of the x-ray detector (4) used to image the molar region (M) of the focal curve (F) is different than the gain of the x-ray detector (4) used to image the incisal region (I).

8. The computer implemented method according to any one of claims 1 to 7, **characterized in that** in the acquisition step, the readout region (R) of the x-ray detector (4) is continuously varied in between 50 to 500 pixels in the width direction, wherein the detector (4) has a native pixel size of 50 to 240 micrometers.

9. The computer implemented method according to any one of claims 1 to 8, **characterized in that** in the acquisition step, the center point of the width of the x-ray aperture (13) relative to the x-ray detector (4) is continuously varied such that the beam direction passing from the x-ray source to the center point of the redout region (R) remains orthoradial to the focal curve (F) or as close as possible to being orthoradial to the focal curve (F).

10. An X-ray system (1) for generating a panoramic image of a patient (P), comprising:
an x-ray source (3);
an x-ray detector (4);
an x-ray collimator (12), wherein the x-ray collimator (12) is positioned between the patient's head and the x-ray source (3), wherein the collimator (12) has an x-ray aperture (13) to restrict the x-ray cone beam angle, wherein the width of the x-ray aperture (13) is adjustable for varying the x-ray cone beam angle; and
a control means configured to carry out the method according to any one of the preceding method claims.

11. A computer program comprising computer-readable code which, when executed by a computer-assisted x-ray system (1), causes the said system to perform the method of any one of the preceding method claims.

12. A computer readable storage means storing the computer program according to claim 11.
